(19) Europäisches Patentamt
European Patent Office
Office européen des brevets

(11) **EP 4 271 351 B1**

(12) **EUROPEAN PATENT SPECIFICATION**

(45) Date of publication and mention
of the grant of the patent:
**24.07.2024 Bulletin 2024/30**

(21) Application number: **21840038.0**

(22) Date of filing: **21.12.2021**

(51) International Patent Classification (IPC):
*A61K 8/23* (2006.01)    *A61K 8/49* (2006.01)
*A61K 8/73* (2006.01)    *A61K 8/81* (2006.01)
*A61Q 5/00* (2006.01)

(52) Cooperative Patent Classification (CPC):
**A61K 8/8158; A61K 8/23; A61K 8/49;**
**A61K 8/4946; A61K 8/737; A61Q 5/006**

(86) International application number:
**PCT/EP2021/086928**

(87) International publication number:
**WO 2022/144226 (07.07.2022 Gazette 2022/27)**

(54) **HAIR CARE COMPOSITION**

HAARPFLEGEZUSAMMENSETZUNG

COMPOSITION DE SOINS CAPILLAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.01.2021 PCT/CN2021/070178**
**05.02.2021 EP 21155345**

(43) Date of publication of application:
**08.11.2023 Bulletin 2023/45**

(73) Proprietors:
• **Unilever IP Holdings B.V.**
**3013 AL Rotterdam (NL)**
Designated Contracting States:
**AL AT BE BG CH CZ DK EE ES FI FR GR HR HU
IS LI LT LU LV MC MK NL NO PL PT RO SE SI SK
SM**
• **Unilever Global IP Limited**
**Wirral, Merseyside CH62 4ZD (GB)**
Designated Contracting States:
**CY DE GB IE IT MT RS TR**

(72) Inventors:
• **LIU, Jingjing**
**Shanghai 200335 (CN)**
• **PI, Yingying**
**Shanghai 200335 (CN)**

(74) Representative: **Mathai, Neenu Grace**
**Unilever Patent Group**
**Bronland 14**
**6708 WH Wageningen (NL)**

(56) References cited:
**WO-A1-2021/013476**

• **DATABASE GNPD [online] MINTEL; 24 October
2019 (2019-10-24), ANONYMOUS: "Anti-Dandruff
Amino Acid Shampoo", XP055827701, retrieved
from
https://www.gnpd.com/sinatra/recordpage/6980
463/ Database accession no. 6980463**
• **DATABASE GNPD [online] MINTEL; 27 March
2019 (2019-03-27), ANONYMOUS: "Alguemarine
Strengthen & Repair Shampoo for Man",
XP055672405, retrieved from
https://www.gnpd.com/sinatra/recordpage/6435
423/ Database accession no. 6435423**

**Description**

**Technical Field of the Invention**

This invention relates to hair care compositions, more particularly to hair care compositions comprising anti-dandruff agents and a combination of copolymer comprising acrylamidopropyltrimonium chloride and cationic guar polymer.

**Background of the Invention**

Hair care compositions generally provide cleansing or conditioning benefits or a combination of the two. Such compositions typically comprise one or more cleansing surfactants which generally aid in cleaning the hair and the scalp free of undesirable soil, particles and fatty matter.

Dandruff control is an important aspect of hair cleansing and care. Anti-dandruff benefit has been provided through hair care compositions including shampoos and hair conditioners. Dandruff is an issue that affects many people globally. The condition is manifested by the shedding of clumps of dead skin cells from the scalp. These are white in colour and provide an aesthetically displeasing appearance. A factor that contributes to dandruff is certain members of the Malassezia yeasts. To combat these, anti-dandruff products have included certain anti-dandruff agents which have anti-fungal activity, for example, piroctone olamine (octopirox®), selenium sulfide or combinations thereof. These anti-dandruff agents remove (or at least reduce the level of) the Malassezia from the scalp and provide moderately effective treatment of the dandruff condition. Such a product performs as a hair cleansing shampoo or as a hair conditioner while mitigating the causes of dandruff.

Mintel GNPD, Record ID 6980463, "Anti-Dandruff Amino Acid Shampoo", Oct 2019 shows an anti-dandruff shampoo comprising piroctone olamine, a copolymer comprising acrylamidopropyltrimonium chloride and a cationic guar polymer.

However, many anti-dandruff products do not provide sufficient anti-dandruff agent deposition onto scalp. Those anti-dandruff agents do not strongly adhere to scalp surfaces and are easily rinsed away during hair wash or shower, therefore provide little or no anti-dandruff efficacy. Consequently, needs exist for a hair care composition which provides improved deposition of anti-dandruff agents to maximize the effectiveness of such anti-dandruff agents.

The present inventors have now found unexpectedly the combination of copolymers comprising acrylamidopropyltrimonium chloride and cationic guar polymer, can enhance deposition of anti-dandruff agents, while delivering the desired sensory experience.

**Tests and Definitions**

Hair Care Composition

"Hair care composition", as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and rinse-off shampoos, conditioners, shower gels, or toilet bar. The composition of the present invention is preferably a rinse-off composition, especially preferred being a shampoo or a conditioner and most preferably a shampoo.

Cationic Charge Density

"Cationic charge density", as used herein, refers to the number of cationic charges per weight unit of a given polymer. Cationic charge density can be calculated from the degree of substitution as described in WO 2013/011122, especially page 8 lines 8-17. For example, for cationic guar polymer obtained by reacting with 2,3-epoxypropyltrimethylammonium chloride, the cationic charge density may be calculated from the degree of substitution using the following equation:

$$\text{Cationic charge density in milliequivalents per gram (meq/g)} = \frac{DS \times 1000}{162 + 151 \times DS}$$

Water-Insoluble

"Water-insoluble", as used herein, refers to the solubility of a material in water at 25°C and atmospheric pressure being 0.1% by weight or less.

Molecular Weight

"Molecular weight", as used herein, refers to the weight average molecular mass of a given polymer. The weight average molecular weight (WAVG MW) of a given polymer is determined by SEC (Size Exclusion Chromatography) analysis using absolute calibration (universal calibration). Polysaccharide standards pulluan and dextran were used for calibration.

Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

**Summary of the Invention**

In a first aspect, the present invention is directed to a hair care composition comprising:

(a) an anti-dandruff agent selected from piroctone olamine, climbazole, ketoconazole, selenium sulfide and mixtures thereof;
(b) a copolymer comprising acrylamidopropyltrimonium chloride; and
(c) a cationic guar polymer having a molecular weight of from 0.3 to 2.5 million Dalton and a cationic degree of substitution of from 0.05 to 0.4;

wherein the weight ratio of the amount of the copolymer to the amount of the cationic guar polymer is in the range of from 1:10 to 10:1.

In a second aspect, the present invention is directed to a non-therapeutic method of depositing anti-dandruff agents onto scalp comprising the step of applying the hair care composition of any embodiment of the first aspect of this invention onto scalp surfaces of an individual. The method is preferably for non-therapeutic benefits.

In a third aspect, the present invention is directed to use of combination of acrylamidopropyltrimonium chloride/acrylamide copolymer and cationic guar polymer for enhancing deposition of anti-dandruff agents onto scalp.

All other aspects of the present invention will more readily become apparent upon considering the detailed description and examples which follow.

**Detailed Description**

Copolymer comprising acrylamidopropyltrimonium chloride

The copolymer suitable for use in compositions of the present invention comprises acrylamidopropyltrimonium chloride. Preferably, the copolymer also comprises acrylamide in addition to the acrylamidopropyltrimonium chloride. Particularly preferred copolymer is a copolymer of acrylamidopropyltrimonium chloride and acrylamide.

The copolymer according to the present invention preferably has a charge density of from 1.0 to 3.0 meq per gram (meq/g), more preferably from 1.1 to 2.5 meq/g, more preferably still from 1.2 to 2.5 meq/g and most preferably from 1.3 to 2.5 meq/g. The copolymer preferably has a molecular weight of from 100,000 to 3,000,000 gram per mole (g/mol), more preferably from 500,000 to 2,800,000 g/mol, more preferably still from 800,000 to 2,500,000 g/mol, most preferably from 1,000,000 to 2,500,000 g/mol. An example of a suitable copolymer is commercially available from Ashland under the trade name N-Hance SP-100®. N-Hance SP-100® has a charge density of from 1.8 to 2.2 meq/g and a molecular weight of from 1,800,000 to 2,200,000 g/mol.

The hair care composition of the present invention typically comprises the copolymer in an amount of from 0.001 to 2%, more preferably from 0.01 to 1%, even more preferably from 0.01 to 0.8% and most preferably from 0.05 to 0.5%, based on total weight of the hair care composition and including all ranges subsumed therein.

Cationic guar polymer

The composition also comprises cationic modified guar polymer having a molecular weight of from 0.3 to 2.5 million Dalton and a cationic degree of substitution of from 0.05 to 0.4.

It is preferred that the cationic modified guar polymer has a molecular weight of from 1.0 to 2.3 million Dalton, more preferably from 1.0 to 1.5 million Dalton.

It is preferred that the cationic guar polymer has a cationic degree of substitution of from 0.1 to 0.3, more preferably from 0.16 to 0.2.

It is most preferred that cationic modified guar polymer has a molecular weight of from 1.0 million to 1.5 million Dalton and a cationic degree of substitution (DS) of from 0.16 to 0.20. This DS value corresponds to a charge density of from 0.85 to 1.05.

Cationic guar polymer is preferably guar hydroxypropyltrimonium chloride. Guar polymer predominantly contains galactomannan polymer chains. This polymer is available at various molecular weights and degree of cationic substitutions depending on how much the guar has been hydrolysed and cationised.

Generally for cationic polysaccharide polymers, the hydroxyl groups of the non-modified monomeric sugar ring units are the sites for cationic substitution. Degree of substitution (DS) is typically in the range from 0 to 3 due to the fact that the monomeric sugar unit of most polysaccharide has in average three hydroxyl groups available for substitution. In addition to the DS, the cationic charge on polymers can also be quantified as cationic charge density. DS has previously been determined by different methods. For example, the cationic charge density of the polymer has in some cases been calculated based on a percent nitrogen content determined via the Kjeldahl method as described in US Pharmacopoeia under chemical tests for nitrogen determination and is expressed in milliequivalents (meq) per gram. The cationic charge density of the polymer in the present invention is, however, calculated from the degree of substitution, which is measured by 1H NMR in a solvent of deuterium oxide ($D_2O$) and deuterium chloride (DCl) mixture.

In many cases the DS obtained from 1H NMR measurement may not be suitable to be compared with that obtained from Kjeldahl method, due to the fact that the two methods are influenced by different factors.

In the wide spectrum of molecular weights of guars available, the cationic guar for use in the present invention has a molecule weight which is considered a 'medium' molecular weight. In the wide range of charge densities, the above range for use in the present invention is considered a 'medium' range. The cationic modified guar deposition polymer is preferably present in an amount of from 0.001 to 2%, more preferably from 0.01 to 1%, in 0.04 to 0.5%, more preferably 0.08 to 0.25% by weight of the composition.

The amount of the copolymer to the amount of the cationic guar polymer in the composition of the present invention is in the range of from 1:10 to 10:1, preferably from 1:5 to 5:1, more preferably 1:3 to 3:1.

Antidandruff agents

The hair care composition comprises anti-dandruff agents, which are compounds that are active against dandruff and are typically anti-microbial agents and preferably anti-fungal agents. Suitable anti-dandruff agents that may be used in this invention are selected from piroctone olamine (octopirox®), climbazole, ketoconazole, selenium sulfide and mixtures thereof. In a preferred embodiment, the antidandruff agent is selected from piroctone olamine (octopirox®) and climbazole. In an especially preferred embodiment, the anti-dandruff agent is piroctone olamine.

Typically, the hair care composition of the invention comprises the anti-dandruff agent in an amount of from 0.01 to 10%, more preferably from 0.01 to 5%, more preferably still from 0.05 to 2%, and most preferably from 0.05 to 1.5%, based on total weight of the hair care composition and including all ranges subsumed therein.

It has been found that the combination of copolymer and cationic guar polymer of the present invention unexpectedly enhances deposition of anti-dandruff agents. It is preferred that the composition comprises the copolymer and the anti-dandruff agent in a weight ratio of from 1:10 to 1:1, preferably from 1:5 to 1:1, and most preferably from 1:4 to 1:1.5.

The pH of the composition is preferably equal to or higher than 4.0, more preferably in the range of 4.0 to 7.0.

In addition to the copolymer, it is preferable that the hair care composition also comprises other cationic polymers. Suitable cationic polymers may be homopolymers or be formed from two or more types of monomers. The molecular weight of the polymer will generally be between 5,000 and 10,000,000 g/mol, typically at least 10,000 g/mol and preferably from 100,000 to 2,000,000 g/mol. The polymers will have cationic nitrogen containing groups such as quaternary ammonium or protonated amino groups, or a mixture thereof. The cationic nitrogen containing group will generally be present as a substituent on a fraction of the total monomer units of the cationic polymer. Thus when the polymer is not a homopolymer it can contain spacer non-cationic monomer units. The ratio of the cationic to non-cationic monomer units is selected to give a polymer having a cationic charge density in the required range.

Suitable cationic polymers include, for example, copolymers of vinyl monomers having cationic amine or quaternary ammonium functionalities with water soluble spacer monomers such as (meth)acrylamide, alkyl and dialkyl (meth) acrylamides, alkyl (meth)acrylate, vinyl caprolactone and vinyl pyrrolidine. The alkyl and dialkyl substituted monomers preferably have $C_1$-$C_7$ alkyl groups, more preferably $C_1$-$C_3$ alkyl groups. Other suitable spacers include vinyl esters, vinyl alcohol, maleic anhydride, propylene glycol and ethylene glycol.

Preferably, the cationic polymer is a cationic polysaccharide polymer such as cationic guar, cationic starch, and cationic cellulose. Suitably, such cationic polysaccharide polymers have a molecular weight of from 100,000 g/mol to 2,300,000 g/mol, more preferably from 150,000 g/mol to 2,000,000 g/mol. Such cationic polysaccharide polymers preferably have a cationic charge density from 0.1 to 4 meq/g.

Cationic polysaccharide polymers suitable for use in compositions of this invention include those represented by the general formula:

$$A\text{-}O\text{-}[R^1\text{-}N^+(R^2)(R^3)(R^4)X^-]$$

wherein: A is an anhydroglucose residual group, such as a starch or cellulose anhydroglucose residual. $R^1$ is an alkylene, oxyalkylene, polyoxyalkylene, or hydroxyalkylene group, or combination thereof. $R^2$, $R^3$ and $R^4$ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in $R^2$, $R^3$ and $R^4$) is preferably about 20 or less, and X is an anionic counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418) and copolymers of etherified cellulose and starch (e.g. as described in U.S. Patent 3,958,581).

When used, the cationic polymer will generally be present in the hair care composition of the present invention in an amount of from 0.001 to 1% by weight of the hair care composition, more preferably from 0.01 to 0.5%, and most preferably from 0.03 to 0.3%, based on total weight of the hair care composition and including all ranges subsumed therein.

The hair care composition may additionally comprise a conditioning agent to provide conditioning benefit. Preferably, the hair care composition comprises discrete dispersed droplets of a water-insoluble conditioning agent, which has a mean droplet diameter ($D_{3,2}$) of less than 15 microns, preferably less than 10 microns, more preferably less than 5 microns, most preferably less than 3 microns. The mean droplet diameter ($D_{3,2}$) of a water-insoluble conditioning agent may be measured by means of a laser light scattering technique, for example using a 2600D Particle Sizer from Malvern Instruments.

The water-insoluble conditioning agent may include non-silicone conditioning agent comprising non-silicone oily or fatty materials such as hydrocarbon oils, fatty esters and mixtures thereof. Preferably, the water-insoluble conditioning agent is emulsified silicone oil.

Suitable silicones include polydiorganosiloxanes, in particular polydimethylsiloxanes which have the CTFA designation dimethicone. Also suitable for use in compositions of this invention (particularly shampoos and conditioners) are polydimethyl siloxanes having hydroxyl end groups, which have the CTFA designation dimethiconol. Also suitable for use in compositions of this invention are silicone gums having a slight degree of crosslinking, as are described for example in WO 96/31188. Preferably, the silicone oil comprises dimethicone, dimethiconol or a mixture thereof.

Suitable emulsified silicones for use in the hair care compositions of this invention are available as pre-formed silicone emulsions from suppliers of silicones such as Dow Corning and GE silicones. The use of such pre-formed silicone emulsion is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Examples of suitable pre-formed silicone emulsions include DC1785, DC1788, DC7128, all available from Dow Corning. These are emulsions of dimethiconol/dimethicone.

Another class of silicones which may be used are functionalized silicones such as amino functional silicones, meaning a silicone containing at least one primary, secondary or tertiary amine group, or a quaternary ammonium group. Examples of suitable amino functional silicones include polysiloxanes having the CTFA designation "amodimethicone."

The water-insoluble conditioning agent is generally present in hair care composition of this invention in an amount from 0.05 to 15%, preferably from 0.1 to 10%, more preferably from 0.5 to 8%, most preferably from 1 to 5%, based on total weight of the hair care composition and including all ranges subsumed therein.

In a preferred embodiment, the hair care composition is a shampoo. Thus in a preferred embodiment the composition comprises a cleansing surfactant. Non-limiting examples of suitable anionic cleansing surfactants are alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule. Typical anionic cleansing surfactants for use in compositions of the invention include, but not limited to, sodium oleyl succinate, ammonium lauryl sulphosuccinate, sodium lauryl sulphate, sodium lauryl ether sulphate, sodium lauryl ether sulphosuccinate, ammonium lauryl sulphate, sodium dodecylbenzene sulphonate, triethanolamine dodecylbenzene sulphonate, sodium cocoyl isethionate, sodium lauryl isethionate, lauryl ether carboxylic acid, sodium N-lauryl sarcosinate or mixtures thereof. Preferred anionic cleansing surfactants are the alkyl sulphates and alkyl ether sulphates. Preferred alkyl sulphates are $C_{8-18}$ alky sulphates, more preferably $C_{12-18}$ alkyl sulphates, preferably in the form of a salt with a solubilising cation such as sodium, potassium, ammonium or substituted ammonium. Examples are sodium lauryl sulphate (SLS) or sodium dodecyl sulphate (SDS). It is particularly preferred that the anionic cleansing surfactant is alkyl ether sulphate. Preferred alkyl ether sulphates are those having the formula: $RO(CH_2CH_2O)_nSO_3M$; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulphate (SLES). Preferred alkyl ether sulphate is sodium lauryl ether sulphate having an average degree of ethoxylation of from 0.5 to 3, preferably from 1 to 3, more preferably from 2 to 3.

The anionic cleansing surfactants are typically present in hair care composition of the present invention at a level of from 0.5 to 45%, more preferably from 1.5 to 35% and most preferably from 5 to 20%, based on total weight of the hair care composition and including all ranges subsumed therein.

The composition as per the invention optionally and preferably additionally comprises co-surfactants such as amphoteric and zwitterionic surfactants to provide mildness to the composition. Suitable examples include alkyl amine oxides, alkyl betaines, alkyl amidopropyl betaines, alkyl sulphobetaines (sultaines), alkyl amphoacetates, alkyl amphopropionates, alkyl amidopropyl hydroxysultaines, wherein the alkyl group has from 8 to 19 carbon atoms. Preferably, the co-surfactant is a betaine surfactant. Cocamidopropyl betaine (CAPB) is particularly preferred.

When used, the co-surfactant typically makes up from 0.1 to 15%, more preferably from 0.5 to 8% and most preferably from 0.5 to 4% by weight of the hair care composition, based on total weight of the hair care composition and including all ranges subsumed therein.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

The suspending agent is generally present in hair care composition of this invention in an amount of from 0.1 to 10%, more preferably from 0.2 to 6%, and most preferably from 0.3 to 4%, based on total weight of the hair care composition and including all ranges subsumed therein.

Preservatives may also be incorporated into the hair care composition of this invention to protect against the growth of potentially harmful microorganisms. Suitable traditional preservatives include alkyl esters of parahydroxybenzoic acid, hydantoin derivatives, propionate salts, and a variety of quaternary ammonium compounds. Illustrative yet non-limiting examples of the types of preservatives that may be used in this invention include, for examples, phenoxyethanol, sodium salicylate, methyl paraben, butyl paraben, propyl paraben, diazolidinyl urea, sodium dehydroacetate, benzyl alcohol, sodium benzoate, iodopropynyl butylcarbamate, caprylyl glycol, disodium EDTA or mixtures thereof. In an especially preferred embodiment, the preservative is sodium benzoate, phenoxyethanol, sodium salicylate or a mixture thereof. Preservatives are preferably employed in amounts ranging from 0.01 to 2% by weight of the hair care composition.

The hair care composition of the present invention may contain other ingredients which are common in the art to enhance physical properties and performances. Suitable ingredients include but are not limited to fragrance, dyes and pigments, pH adjusting agents, pearlescers or opacifiers, viscosity modifiers, thickeners, and natural hair nutrients such as botanicals, fruit extracts, sugar derivatives and amino acids.

Method and Use

The present invention also provides for a method of depositing anti-dandruff agents onto scalp comprising the step of applying the hair care composition according to any of the preceding claims onto scalp surfaces of an individual followed by rinsing the surfaces with water. The anti-dandruff agent is selected from piroctone olamine, climbazole, ketoconazole, selenium sulfide and mixtures thereof. It is preferred the anti-dandruff agent is piroctone olamine. The method is non-therapeutic in nature. Preferably it is for cosmetic purpose.

The present invention also provides for use of combination of acrylamidopropyltrimonium chloride/acrylamide copolymer and cationic guar polymer for enhancing deposition of anti-dandruff agents onto scalp, wherein the anti-dandruff agent is selected from piroctone olamine, climbazole, ketoconazole, selenium sulfide and mixtures thereof. It is preferred that the cationic guar polymer has a molecular weight of from 0.3 to 2.5 million Dalton and a cationic degree of substitution of from 0.05 to 0.4. It is preferred the anti-dandruff agent is piroctone olamine. The use is non-therapeutic in nature, preferably cosmetic in nature.

Mode of Use

The compositions of the invention are primarily intended for topical application to scalp and/or at least a portion of the hair of an individual, either in rinse-off or leave-on compositions, preferably in rinse-off compositions like shampoos.

When the composition is a shampoo, it is topically applied to the hair and then massaged into the hair and scalp. Then it is rinsed with water prior to drying the hair.

The invention will be further illustrated by the following, non-limiting Examples, in which all percentages quoted are by weight based on total weight unless otherwise stated.

The examples are intended to illustrate the invention and are not intended to limit the invention to those examples per se.

**Examples**

Example 1

This example demonstrated the combination of copolymer and cationic guar polymer can affect the deposition of anti-dandruff agent onto scalp. All ingredients are expressed by weight percent of the total formulation, and as level of active ingredient.

TABLE 1

| Ingredient | Samples | |
|---|---|---|
| | A | 1 |
| Water | Balance | Balance |
| Sodium lauryl ether sulphate | 9.66 | 9.66 |
| Piroctone olamine (Octopirox®) | 0.30 | 0.30 |
| Disodium EDTA | 0.05 | 0.05 |
| Carbopol 980 | 0.35 | 0.35 |
| Acrylamidopropyltrimonium chloride/acrylamide copolymer[a] | 0.00 | 0.05 |

(continued)

| Ingredient | Samples | |
|---|---|---|
| | **A** | **1** |
| Guar hydroxypropyltrimonium chloride[b] | 0.20 | 0.15 |
| Cocoamidopropyl betaine | 2.10 | 2.10 |
| Perfume | 0.75 | 0.75 |
| Dimethicone | 1.00 | 1.00 |
| Dimethiconol | 1.50 | 1.50 |
| Sodium benzoate | 0.50 | 0.50 |
| Citrate acid | 0.35 | 0.35 |
| Sodium chloride | 0.70 | 0.70 |
| a. Commercial acrylamidopropyltrimonium chloride/acrylamide copolymer under the trade name N-Hance SP100 from Ashland. b. Commercial guar hydroxypropyltrimonium chloride has a DS of 0.16 to 0.20 and a weight average molecular weight from 1.0 to 1.5 million g/mol under the trade name BB-18 from Lamberti. | | |

*Methods*

About 0.2 grams of the test sample was taken on artificial skin (VITRO-SKIN from IMS testing group). This was diluted with 1.8 mL water and rubbed with a plastic rod for 30 seconds. The artificial skin surface was then rinsed twice with water, first time with 4 mL water for 30 second and then again with 4 mL water for 30 seconds. The deposition of piroctone olamine on the skin ($10.75 \text{ cm}^2$ per plate) was measured using HPLC method.

*Results*

The average deposition (of five such experiments) are summarized in Table 2 (error represents standard deviation for duplicate measurements).

TABLE 2

| Samples | A | 1 |
|---|---|---|
| Piroctone olamine deposition ($\mu$g/plate) | $6.26 \pm 0.53$ | $7.09 \pm 0.31$ |

Samples 1 comprising combination of copolymer and cationic guar polymer showed significantly better ($p < 0.05$) deposition of piroctone olamine as compared to samples A (comprising cationic guar polymer only).

Example 2

This example shows the desired sensorial experience can be delivered by the combination of the combination of copolymer and cationic guar polymer. Compositions were prepared according to the formulations detailed in Table 3. All ingredients are expressed by weight percent of the total formulation, and as level of active ingredient.

**TABLE 3**

| Ingredient | Samples | |
|---|---|---|
| | **B** | **2** |
| Water | Balance | Balance |
| Sodium lauryl ether sulphate | 12.66 | 12.66 |
| Piroctone olamine (Octopirox®) | 0.75 | 0.75 |
| Disodium EDTA | 0.05 | 0.05 |

(continued)

| Ingredient | Samples | |
|---|---|---|
| | **B** | **2** |
| Carbopol 980 | 0.35 | 0.35 |
| Acrylamidopropyltrimonium chloride/acrylamide copolymer[a] | 0.20 | 0.05 |
| Guar hydroxypropyltrimonium chloride[b] | 0.00 | 0.15 |
| Cocoamidopropyl betaine | 0.80 | 0.80 |
| Perfume | 0.75 | 0.75 |
| Dimethicone | 1.00 | 1.00 |
| Dimethiconol | 1.50 | 1.50 |
| Sodium benzoate | 0.50 | 0.50 |
| Citrate acid | 0.35 | 0.35 |
| Sodium chloride | 0.70 | 0.70 |
| a. Commercial acrylamidopropyltrimonium chloride/acrylamide copolymer under the trade name N-Hance SP100 from Ashland. b. Commercial guar hydroxypropyltrimonium chloride has a DS of 0.16 to 0.20 and a weight average molecular weight from 1.0 to 1.5 million g/mol under the trade name BB-18 from Lamberti. | | |

*Methods*

This example demonstrates the improved performance of a shampoo according to the invention as determined by a sensory study.

In the salon trial the samples were tested on 36 female respondents. The respondents were evaluated prior to the trial to have the same type of hair (e.g. normal hair, dry hair, greasy hair etc). In a given trial, 18 respondents with normal hair and 18 with dry hair were chosen. The evaluation was done by trained hairdressers with good sensitivity. The evaluation method involved paired comparison with half the head washed with the sample B and the other half of the head washed with the sample 2.

*Results*

The stylists were asked to evaluate the panelists' hair against a range of standard attributes. The results showed that Shampoo 2 was significantly (at least 95% conf.) higher in the following attributes:

Lather properties:

- Speed to lather
- Overall hair foam
- More amount of lather

It can be seen from the results that sample 2 comprising copolymer and cationic guar polymer can enhance the sensorial experience especially foaming/lathering related sensory, compared to sample B comprising copolymer only.

Example 3

This example demonstrated the ratio between copolymer and cationic guar polymer can affect the deposition of anti-dandruff agent onto scalp. Compositions were prepared according to the formulations detailed in Table 4. All ingredients are expressed by weight percent of the total formulation, and as level of active ingredient.

**TABLE 4**

| Ingredient | Samples | | |
|---|---|---|---|
| | C | 3 | 4 |
| Water | Balance | Balance | Balance |
| Sodium lauryl ether sulphate | 9.66 | 9.66 | 9.66 |
| Piroctone olamine (Octopirox®) | 0.30 | 0.30 | 0.30 |
| Disodium EDTA | 0.05 | 0.05 | 0.05 |
| Carbopol 980 | 0.35 | 0.35 | 0.35 |
| Acrylamidopropyltrimonium chloride/acrylamide copolymer[a] | 0.015 | 0.033 | 0.05 |
| Guar hydroxypropyltrimonium chloride[b] | 0.185 | 0.167 | 0.15 |
| Cocoamidopropyl betaine | 2.10 | 2.10 | 2.10 |
| Perfume | 0.75 | 0.75 | 0.75 |
| Dimethicone | 1.00 | 1.00 | 1.00 |
| Dimethiconol | 1.50 | 1.50 | 1.50 |
| Sodium benzoate | 0.50 | 0.50 | 0.50 |
| Citrate acid | 0.35 | 0.35 | 0.35 |
| Sodium chloride | 0.70 | 0.70 | 0.70 |
| Ratio of the copolymer to the cationic guar polymer | 1:12 | 1:5 | 1:3 |
| a. Commercial acrylamidopropyltrimonium chloride/acrylamide copolymer under the trade name N-Hance SP100 from Ashland. <br> b. Commercial guar hydroxypropyltrimonium chloride has a DS of 0.16 to 0.20 and a weight average molecular weight from 1.0 to 1.5 million g/mol under the trade name BB-18 from Lamberti. | | | |

*Methods*

The same deposition evaluation method was used as disclosed in Example 1.

*Results*

The average deposition (of five such experiments) is summarized in Table 5.

**TABLE 5**

| Samples | C | 3 | 4 |
|---|---|---|---|
| Piroctone olamine deposition ($\mu$g/plate) | 6.77 | 7.9 | 7.95 |

Samples 3 and 4 comprising copolymer and cationic guar polymer with ratio within the range of the present invention showed better deposition of piroctone olamine as compared to samples C (with the ration outside the range of the present invention).

Example 4

This example demonstrated the ratio between copolymer and cationic guar polymer can affect sensorial experience upon application. Compositions were prepared according to the formulations detailed in Table 6. All ingredients are expressed by weight percent of the total formulation, and as level of active ingredient.

## TABLE 6

| Ingredient | Samples | |
|---|---|---|
| | D | 5 |
| Water | Balance | Balance |
| Sodium lauryl ether sulphate | 12.66 | 12.66 |
| Piroctone olamine (Octopirox®) | 0.75 | 0.75 |
| Disodium EDTA | 0.05 | 0.05 |
| Carbopol 980 | 0.35 | 0.35 |
| Acrylamidopropyltrimonium chloride/acrylamide copolymer[a] | 0.184 | 0.05 |
| Guar hydroxypropyltrimonium chloride[b] | 0.016 | 0.15 |
| Cocoamidopropyl betaine | 0.80 | 0.80 |
| Perfume | 0.75 | 0.75 |
| Dimethicone | 1.00 | 1.00 |
| Dimethiconol | 1.50 | 1.50 |
| Sodium benzoate | 0.50 | 0.50 |
| Citrate acid | 0.35 | 0.35 |
| Sodium chloride | 0.70 | 0.70 |
| Ratio of the copolymer to the cationic guar polymer | 11.5:1 | 1:3 |

a. Commercial acrylamidopropyltrimonium chloride/acrylamide copolymer under the trade name N-Hance SP100 from Ashland.
b. Commercial guar hydroxypropyltrimonium chloride has a DS of 0.16 to 0.20 and a weight average molecular weight from 1.0 to 1.5 million g/mol under the trade name BB-18 from Lamberti.

*Methods*

A sensory study was used to evaluate the performance as disclosed in Example 2.

*Results*

The stylists were asked to evaluate the panellists' hair against a range of standard attributes. The results showed that Shampoo 5 was significantly (at 99% conf.) higher in the following attributes:
Lather properties:

- Speed to lather
- Overall hair foam
- More amount of lather

It can be seen from the results that sample 5 comprising the copolymer and cationic guar polymer with the ration within the range of the present invention can enhance the sensorial experience especially foaming/lathering related sensory, compared to sample D with weight ratio between the copolymer and cationic guar polymer outside the present invention.

**Claims**

1. A hair care composition comprising:

    a) an anti-dandruff agent selected from piroctone olamine, climbazole, ketoconazole, selenium sulfide and mixtures thereof; and

b) a copolymer comprising acrylamidopropyltrimonium chloride;

c) a cationic guar polymer having a molecular weight of from 0.3 to 2.5 million Dalton and a cationic degree of substitution of from 0.05 to 0.4;

wherein the weight ratio of the amount of the copolymer to the amount of the cationic guar polymer is in the range of from 1:10 to 10:1.

2. A composition as claimed in claim 1 wherein the anti-dandruff agent is piroctone olamine.

3. A composition as claimed in claim 1 or 2 wherein the copolymer is acrylamidopropyltrimonium chloride/acrylamide copolymer.

4. A composition as claimed in any of claims 1 to 3 wherein the cationic guar polymer has a molecular weight of from 1.0 to 2.3 million Dalton.

5. A composition as claimed in any of claims 1 to 4 wherein the cationic guar polymer has a cationic degree of substitution of from 0.1 to 0.3.

6. A composition as claimed in any of claims 1 to 5 wherein the cationic guar polymer is guar hydroxypropyl trimonium chloride.

7. A composition as claimed in any of claims 1 to 6 wherein the composition comprises the copolymer in an amount of from 0.001 to 2% by weight of the composition, preferably from 0.01 to 1%.

8. A composition as claimed in any of claims 1 to 7 wherein the composition comprises the anti-dandruff agent in an amount of from 0.01 to 10% by weight of the composition, preferably from 0.01 to 5%.

9. A composition as claimed in any of claims 1 to 8 wherein the composition comprises the cationic guar polymer in an amount of from 0.01 to 2% by weight of the composition, preferably from 0.04 to 2%.

10. A composition as claimed in any of claims 1 to 9 wherein the weight ratio of the amount of the copolymer to the amount of the cationic guar polymer is in the range of from 1:5 to 5:1, preferably from 1:3 to 3:1.

11. A composition as claimed in any of claims 1 to 10 wherein the composition comprises a cleansing surfactant, preferably sodium lauryl ether sulphate.

12. A composition as claimed in any of claims 1 to 11 wherein the composition comprises a conditioning agent, preferably silicone oil, more preferably dimethicone, dimethiconol or a mixture thereof.

13. A composition as claimed in any of claims 1 to 12 wherein the composition is a shampoo.

14. A non-therapeutic method of depositing anti-dandruff agents onto scalp comprising the step of applying the hair care composition as claimed in any of claims 1 to 13 onto scalp surfaces of an individual followed by rinsing the surfaces with water.

15. Use of combination of acrylamidopropyltrimonium chloride/acrylamide copolymer and cationic guar polymer for enhancing deposition of anti-dandruff agents onto scalp, wherein the anti-dandruff agent is selected from piroctone olamine, climbazole, ketoconazole, selenium sulfide and mixtures thereof.


**Patentansprüche**

1. Haarpflegezusammensetzung, umfassend:

a) ein Antischuppenmittel, ausgewählt unter Piroctonolamin, Climbazol, Ketoconazol, Selensulfid und Mischungen davon; und

b) ein Copolymer, umfassend Acrylamidopropyltrimoniumchlorid;

c) ein kationisches Guarpolymer mit einem Molekulargewicht von 0,3 bis 2,5 Millionen Dalton und einem kationischen Substitutionsgrad von 0,05 bis 0,4;

wobei das Gewichtsverhältnis der Menge des Copolymers zu der Menge des kationischen Guarpolymers in dem Bereich von 1:10 bis 10:1 liegt.

2. Zusammensetzung, wie im Anspruch 1 beansprucht, wobei das Antischuppenmittel Piroctonolamin ist.

3. Zusammensetzung, wie im Anspruch 1 oder 2 beansprucht, wobei das Copolymer Acrylamidopropyltrimoniumchlorid/Acrylamid-Copolymer ist.

4. Zusammensetzung, wie in einem der Ansprüche 1 bis 3 beansprucht, wobei das kationische Guarpolymer ein Molekulargewicht von 1,0 bis 2,3 Millionen Dalton aufweist.

5. Zusammensetzung, wie in einem der Ansprüche 1 bis 4 beansprucht, wobei das kationische Guarpolymer einen kationischen Substitutionsgrad von 0,1 bis 0,3 aufweist.

6. Zusammensetzung, wie in einem der Ansprüche 1 bis 5 beansprucht, wobei das kationische Guarpolymer Guarhydroxypropyltrimoniumchlorid ist.

7. Zusammensetzung, wie in einem der Ansprüche 1 bis 6 beansprucht, wobei die Zusammensetzung das Copolymer in einer Menge von 0,001 bis 2 Gewichts-% der Zusammensetzung, bevorzugt von 0,01 bis 1 Gewichts-%, umfasst.

8. Zusammensetzung, wie in einem der Ansprüche 1 bis 7 beansprucht, wobei die Zusammensetzung das Antischuppenmittel in einer Menge von 0,01 bis 10 Gewichts-% der Zusammensetzung, bevorzugt von 0,01 bis 0,5 Gewichts-%, umfasst.

9. Zusammensetzung, wie in einem der Ansprüche 1 bis 8 beansprucht, wobei die Zusammensetzung das kationische Guarpolymer in einer Menge von 0,01 bis 2 Gewichts-% der Zusammensetzung, bevorzugt von 0,04 bis 2 Gewichts-%, umfasst.

10. Zusammensetzung, wie in einem der Ansprüche 1 bis 9 beansprucht, wobei das Gewichtsverhältnis der Menge des Copolymers zu der Menge des kationischen Guarpolymers in dem Bereich von 1:5 bis 5:1, bevorzugt von 1:3 bis 3:1, liegt.

11. Zusammensetzung, wie in einem der Ansprüche 1 bis 10 beansprucht, wobei die Zusammensetzung ein Reinigungstensid, bevorzugt Natriumlaurylethersulfat, umfasst.

12. Zusammensetzung, wie in einem der Ansprüche 1 bis 11 beansprucht, wobei die Zusammensetzung ein Konditionierungsmittel, bevorzugt Silikonöl, bevorzugter Dimethicon, Dimethiconol oder eine Mischung davon, umfasst.

13. Zusammensetzung, wie in einem der Ansprüche 1 bis 12 beansprucht, wobei die Zusammensetzung ein Shampoo ist.

14. Nicht-therapeutisches Verfahren zum Auftragen von Antischuppenmitteln auf die Kopfhaut, umfassend den Schritt des Auftragens der Haarpflegezusammensetzung, wie in einem der Ansprüche 1 bis 13 beansprucht, auf die Kopfhautoberfläche einer Person mit anschließendem Abspülen der Oberflächen mit Wasser.

15. Verwendung der Kombination aus Acrylamidopropyltrimoniumchlorid/ Acrylamid-Copolymer und kationischem Guarpolymer zur Verbesserung der Ablagerung von Antischuppenmitteln auf der Kopfhaut, wobei das Antischuppenmittel unter Piroctonolamin, Climbazol, Ketoconazol, Selensulfid und Mischungen davon ausgewählt ist.

**Revendications**

1. Composition de soins capillaires comprenant:

a) un agent antipelliculaire choisi parmi la piroctone olamine, le climbazole, le kétoconazole, le sulfure de sélénium et leurs mélanges; et

b) un copolymère comprenant du chlorure d'acrylamidopropyltrimonium;

c) un polymère de guar cationique ayant un poids moléculaire de 0,3 à 2,5 millions de Daltons et un degré de substitution cationique de 0,05 à 0,4;

dans laquelle le rapport pondéral de la quantité de copolymère à la quantité de polymère de guar cationique est dans la plage de 1:10 à 10:1.

2. Composition selon la revendication 1, dans laquelle l'agent antipelliculaire est la piroctone olamine.

3. Composition selon la revendication 1 ou 2, dans laquelle le copolymère est un copolymère chlorure d'acrylamidopropyltrimonium/acrylamide.

4. Composition selon l'une quelconque des revendications 1 à 3, dans laquelle le polymère de guar cationique a un poids moléculaire de 1,0 à 2,3 millions de Daltons.

5. Composition selon l'une quelconque des revendications 1 à 4, dans laquelle le polymère de guar cationique a un degré de substitution cationique de 0,1 à 0,3.

6. Composition selon l'une quelconque des revendications 1 à 5, dans laquelle le polymère de guar cationique est le chlorure d'hydroxypropyltrimonium de guar.

7. Composition selon l'une quelconque des revendications 1 à 6, dans laquelle la composition comprend le copolymère en une quantité de 0,001 à 2 % en poids de la composition, de préférence de 0,01 à 1 %.

8. Composition selon l'une quelconque des revendications 1 à 7, dans laquelle la composition comprend l'agent antipelliculaire en une quantité de 0,01 à 10 % en poids de la composition, de préférence de 0,01 à 5 %.

9. Composition selon l'une quelconque des revendications 1 à 8, dans laquelle la composition comprend le polymère de guar cationique en une quantité de 0,01 à 2 % en poids de la composition, de préférence de 0,04 à 2 %.

10. Composition selon l'une quelconque des revendications 1 à 9, dans laquelle le rapport pondéral de la quantité de copolymère à la quantité de polymère de guar cationique est dans la plage de 1:5 et 5:1, de préférence de 1:3 à 3:1.

11. Composition selon l'une quelconque des revendications 1 à 10, dans laquelle la composition comprend un tensioactif nettoyant, de préférence du lauryléther sulfate de sodium.

12. Composition selon l'une quelconque des revendications 1 à 11, dans laquelle la composition comprend un agent revitalisant, de préférence de l'huile de silicone, de préférence encore de la diméthicone, du diméthiconol ou un mélange de ceux-ci.

13. Composition selon l'une quelconque des revendications 1 à 12, dans laquelle la composition est un shampooing.

14. Procédé non thérapeutique de dépôt d'agents antipelliculaires sur le cuir chevelu comprenant l'étape d'application de la composition de soins capillaires selon l'une quelconque des revendications 1 à 13 sur des surfaces du cuir chevelu d'un individu, suivie du rinçage des surfaces à l'eau.

15. Utilisation d'une combinaison de copolymère chlorure d'acrylamidopropyltrimonium/acrylamide et de polymère de guar cationique pour améliorer le dépôt d'agents antipelliculaires sur le cuir chevelu, dans laquelle l'agent antipelliculaire est choisi parmi la piroctone olamine, le climbazole, le kétoconazole, le sulfure de sélénium et leurs mélanges.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013011122 A
- US 3962418 A
- US 3958581 A
- WO 9631188 A